# EUROPEAN PATENT SPECIFICATION

(11) **EP 2 408 319 B1**
(45) Date of publication and mention of the grant of the patent: **10.08.2016**
(21) Application number: 10711818.4
(22) Date of filing: 19.03.2010
(51) Int. Cl.: A61K 36/74, A61K 8/97, A61Q 19/00, A23L 29/206, A61Q 1/06

(54) **A PROCESS FOR OBTAINING INSOLUBLE SUBSTANCES FROM GENIPAP-EXTRACT PRECIPITATES, SUBSTANCES FROM GENIPAP-EXTRACT PRECIPITATES AND THEIR USES**
VERFAHREN ZUR GEWINNUNG VON UNLÖSLICHEN SUBSTANZEN AUS GENIPAP-EXTRAKT-NIEDERSCHLÄGEN, SUBSTANZEN AUS GENIPAP-EXTRAKT-NIEDERSCHLÄGEN UND IHRE VERWENDUNGEN
PROCÉDÉ POUR OBTENIR DES SUBSTANCES INSOLUBLES À PARTIR DE PRÉCIPITÉS D'EXTRAITS DE GÉNIPAPE, SUBSTANCES OBTENUES À PARTIR DE PRÉCIPITÉS D'EXTRAITS DE GÉNIPAPE ET LEURS UTILISATIONS

(30) Priority: 20.03.2009 FR 0951813
(43) Date of publication of application: 25.01.2012
(73) Proprietor: Natura Cosméticos S.A., 06882-700 Itapecerica da Serra- SP (BR)
(72) Inventor: ROESLER, Roberta, 13066-022 Campinas - SP (BR); FERRARI, Cintia Rosa, 06390-500 Carapicuíba - SP (BR); DE SOUZA FERREIRA, Cinthia Fernanda, 09811-050 São Bernardo do Campo - SP (BR)
(74) Representative: Pearson, Samuel John
(86) International application number: PCT/BR2010/000082
(87) International publication number: WO 2010/105320

(56) References cited:
- WO-A1-2009/120579
- DE-A1-102005 034 003
- US-A- 4 247 698
- RAJENDRO SINGH 1 N ET AL: "Traditional knowledge and natural dyeing system of Manipur - with special reference to Kum dye" INDIAN JOURNAL OF TRADITIONAL KNOWLEDGE, RESOURCES, NEW DELHI, NEW DELHI - INDIA, vol. 8, no. 1, 1 January 2009 (2009-01-01), pages 84-88, XP018024075 ISSN: 0972-5938

## Description

The present invention relates to the precipitation of genipap (*Genipa americana*) extract for obtaining insoluble substances for application in cosmetic, pharmaceutical, textile, food, paint and varnish compositions.

### BACKGROUND OF THE INVENTION

In Brazil, the plant used in the present invention is known as "jenipapo, jenipá", among others (in English "genipap" or "genipapo"). Genipap belongs to the family *Rubiacea,* genus *Genipa,* species *Americana.*

The use of the genipap fruit is quite comprehensive. The ripe fruit is used more frequently in the form of liquor and sweets. In the Northeast of Brazil, genipap juice is used to fight anemia and as a diuretic against ulcers. In household medicine, the tea made from genipap roots has purgative properties, the smashed seeds induce vomit; the tea from genipap leaves has antidiarrheic effect, among other uses. The unripe fruit produces a dye that is in use since remote times by Indians and produces, by oxidation, a dye with a color going from blue to black, water-soluble, and some alcohols like ethanol and methanol. This dye is much used for dyeing fabrics, ceramics and for obtaining semipermanent tattoos.

In Brazil, the genipap plant occurs in almost every region, mainly in the Southeast, Center-West and North.

The dyeing substance is called "genipina" (genipin) and loses the dyeing effect when the fruit ripens. Thus, the more the genipap fruit is unripe the more intense the color will be. The dye, which is slightly greenish right after extraction, reacts with amino acids in the presence of oxygen, becoming blue or greenish black.

Genipin is an aglycone derived from geniposide, a glycosidic iridoid, which is found in the genipap fruit.

Iridoids are synthesized through mevalonic acid and are known technically as monoterpenoids of [c]-cyclopentane carbonic acid. The aglycones of the glycosidic iridoids have a furan or pyran in their structures.

Genipin is colorless in solution, but it reacts spontaneously with primary amino acids in the presence of oxygen and forms a pigment of bluish black color. From genipin it is possible to obtain other dyeing substances through combination or chemical substitutions.

The tanning agents derived from gallic acid, salts of aluminum salts, calcium and other polyvalent metal salts, like aluminum, iron, zinc, copper, barium, magnesium, manganese, antimony, tin, among others, are widely known in the dyeing industry and pigments as mordants for fixing dyes, acting as a precipitating agent or adsorbent, can be employed for stabilizing natural dyes, the molecules of which, in the presence of light, heat and variation of pH, lose or change their color easily.

At present, due to the begin of the need to replace synthetic dyes and pigments, where many molecules have related adverse reactions, it has become important to find means to stabilize such molecules.

The inventions disclosed in patent documents PI 0005165-9, PI 0402011-1, US 5078750, WO 2002/36364, US 2003/064039, US 2004/076650, US 2003/064039, JP 2000-319120, JP 5-194177, JP 6-056638, JP 7-310023, JP 5-339134, JP 8-301739, JP 2001-122731, JP 8-231353, JP 200-0958859, JP 9-030949, JP 1-124322 and US 2004/0197429 show the obtainment of plant extracts, the stabilization and different application of *Genipa Americana* extract, mainly focusing on therapeutic uses for hair treatments, dyes for dyeing hair and drawings like semi-permanent tattoos. However, all the applications available in the prior art focus on the use of the genipap extract in the water-soluble form and do not use the *Genipa americana* extract in the insoluble form.

Document PI 0005165-9 relates to the obtainment of dyeing extracts and subsequent modification with tannin extract, making the plant extract dye more stable for application in the water-soluble form in cosmetic, food, paint compositions, among others.

Document PI 0402011-1 claims protection for a method of preparing a compound for drawing a non-permanent tattoo, based on the following steps: extracting a determined amount of juice from the Genipa Americana fruit ("Jenipapo"), adding a consistency agent to this juice, adding plant dyes to this mixture, among others. In this case, the compound intended for drawing is also water-soluble.

Document WO 2002/36364 discloses a system of transfer from colored sample/mold onto the skin, comprising, among its ingredients, an adhesive matrix, an adhesive polymer and at least one effective skin coloration agent, which may be a fat-dispersible organic agent from the *Genipa americana* extract, for example.

Document US 5078750 proposes a method of dyeing/coloring hair, which comprises contacting said hair fiber with an iridoidic glycoside, which is geniposidic acid, or an aglycone, which is genipin.

Document US 2003/064039 relates to a composition comprising, in a physiologically acceptable medium, goniochromatic pigments and additinal coloration agents in the proportion of 0.1-2. Among the options of pigments, which can be part of this group of additional coloring agents are the pigments extracted from natural plant sources, as for example *Genipa americana.*

Document US 2004/0766650 claims protection for a composition comprising a physiologically acceptable medium, "interfering" particles that exhibit a coloring effect on the coloration of the composition, in combination with an additional dyeing agent, in an amount sufficient to mask said color imparted by the particles to the preparation. The additional coloring agent must contain in is composition at least one synthetic or organic pigment, which can be extracted from plant sources, such as (water-soluble) *Genipa Americana* extract. Further, it is described that the invention can be applied in hair-makeup compositions.

Document JP 2000-319120 relates to a cosmetic for bath and wash, including a plant constituent, which has properties of retaining the natural skin moisturizing effect. Among the extracts mentioned as option is the huito extract (*Genipa americana).*

Document JP 5-194177 relates to a topical-application agent, which exhibits excellent preventive and chromatosis promoting effects, by stimulating the melanogenesis suppressing action in chromocytes. Said agent has *Genipa ameri*cana extract as its active ingredient.

Document JP 6-056638 relates to an iridoidic hair dye capable of coloring the hairs in a short time and in a safe manner. Said dye can be obtained from a plant belonging to the family *Rubiaceae,* among others, as is the case of geniposide, geniposidic acid and genipin.

Document JP5-339134 relates to a stable hair dye, capable of dyeing the hair promptly in a specific tonality or density, without causing adverse effects in the users thereof. Said dye comprises an aglycone, which is genipin, in combination with an amino acid, like L-arginine, for coloring one's hair in a bluish black tone.

Document JP 8-301739 relates to a composition for the treatment of hair, which contains *Genipa americana* extract as an active dye ingredient, exhibiting an excellent uniformity with regard to the dyeing of white hair.

Document JP 2001-122731 relates to a cosmetic for bath and wash, which includes a vegetable constituent having properties of retaining the natural skin moisturizing effect. Among the extracts mentioned as option is huito extract (*Genipa Americana).*

Document JP 8-231353 relates to a hair tonic, which has the effect of preventing hair loss, besides stimulating the development of hair fiber. Said tonic preferably comprises huito extract (*Genipa americana*) as its active ingredient.

Document JP 2000-095654 relates to a composition of low adhesiveness to hand skin and to hair, but with excellent capability of coloring the hair fibers. The composition is based on a blue pigment solution (genipin) extracted from gardenia, among other ingredients).

Document JP 5058859 relates to a rapid-action dye for skin and hair, based on a combination between geniposides and/or genipins and an aniline derivative.

Document US 2004/0197429 deals with the anti-inflammatory potential (inhibition of COX-2) of some plants, among which are the species belonging to the genus *Genipa,* like *Genipa americana,* for example.

### OBJECTIVES OF THE INVENTION

The present invention has the objective of obtaining insoluble substances from *Genipa americana* extract precipitates and using this precipitate to application, for example, in cosmetic, pharmaceutical, food, textile, paint and varnish compositions, imparting color to the human, animal or vegetable product and/or to the skin and /or to the protein or cellulose material, during the administration thereof.

### SUMMARY OF THE INVENTION

This invention relates to the precipitation of substances from water-soluble genipap extract, among them the dyeing substance genipin, in its insoluble form, using mordants or adsorbent substances. In one aspect, the present invention provides a process for obtaining substances from genipap extract precipitates, characterized by comprising the following steps:
a) obtaining an extract from a genipap fruit;
b) adding a mordent to the extract obtained in (a), wherein the mordent is a polyvalent meal salt selected from the group consisting of aluminum, iron, zinc, copper, calcium, barium, magnesium, manganese, antimony, tin and strontium and/or a natural substrate comprising compounds derived from gallic acid, preferably tannin; and
c) separating the solid substances precipitated from step (b).

The present invention also relates to the precipitated solid substances obtainable from a genipap extract by the above-described process, as well as to cosmetic, pharmaceutical or food compositions containing them and to the uses thereof.

### DETAILED DESCRIPTION OF THE INVENTION

The precipitation process of the present invention includes making a semi-purification and standardization of the genipap extract, and the mordant is selected from polyvalent metal salts selected from the group consisting of aluminum, iron, zinc, copper, calcium, barium, magnesium, manganese, antimony, tin and strontium, more preferably aluminum and calcium, or from natural mordants comprising compounds derived from gallic acid, preferably tannin. In order for the desired precipitation to take place, one should take into consideration the process of purifying the extract, temperature, stirring and ranges of adequate and specific pH for each reactant used and that, therefore, are no limiting parameters of the invention.

Considering that the crystallization and precipitation process is extremely complex, non-colorant compounds present in the extract, such as amino acids, minerals, tannins, sugars, saponines, cumarins, flavonoids, etc., generally play a fundamental role in complexing it with the substrates and the mordents, in order there to be destabilization of the particle, crystallization and, finally, precipitation. Thus, it is important to define the exact degree of purification, so as to obtain a sufficiently standardized and pure precipitate for cosmetic application (without the risk of toxicity, microbial contamination, et.), but, at the same time, with sufficient amounts of non-dyeing compounds for complexation and formation of crystals. In the case of genipap extract, the present invention preferably uses a semi-purified and standardized extract that maintains adequate levels of non-dyeing compounds, indispensable for the complexation and crystallization of the extract. Such non-dyeing compounds can vary in the extract from 5 to 20%, preferably from 10 to 16%, by weight. Additionally, for the color-standardization process one may add compounds like amino acids, in order to balance the initial amount of this compound, which varies depending on the ripening state of the fruit between 100 and 800 ppm according to the extracts obtained during the study.

The process of the invention transforms a water-soluble genipap extract into an insoluble precipitate. Soluble parts are also formed, but they can then be removed from the insoluble product by washing. Preferably, the water-soluble precipitate, when formed, should be removed almost completely.

The substances obtained by precipitating genipap extract according to the invention are insoluble in polar and/or non-polar media. The advantage of the insoluble form is that it can be used as suspensions in water-soluble and liposoluble formulations, as well as in powdered formulation (e.g. shade, blush, etc.). Additionally, the insoluble form exhibits high covering (coloration) of the skin. The water-soluble form of the genipap extract, even in powder (after drying by spray-drier or lyophilization), does not impart coloration with high skin covering.

Depending on the type of final formulation desired, a compositions according to the present invention may use either the insoluble form or the insoluble and water-soluble forms together. In order to obtain the formulation, one does not carry out solubilization, but rather dispersion. Some compositions according to the invention may use only the insoluble extract, and others may use the soluble and insoluble extracts together in order to obtain synergism. It is not possible to obtain adequate and desirable coloration and covering for make-up procedures, without the insoluble form being present.

According to a preferred embodiment, the present invention relates to a process for obtaining substances from genipap extract precipitates, comprising the steps of:
a) obtaining an extract from a genipap fruit; preferably one defines beforeland the ripening degree, considering parameters like texture, diameter and color, which will depend on the specific fruits used;
b) semi-purification of the raw extract and qualification of non-dyeing compounds;
c) standardization of the extract by adding non-dyeing compounds;
d) adding an adsorvent and a mordant to the extract obtained in (c);
e) separating the solid substances precipitated from step (d);
f) washing and adjusting the electric conductivity of the precipitates;
g) drying in an oven; and
h) grinding or sieving for homogenization of the particle size of the dried precipitate obtained in (g).

All the process is carried out under adequate and specific conditions of temperature, stirring an pH, which, however, should be determined according to each specific case and do not represent parameters limitative of the process of the present invention.

According to a preferred embodiment of the invention, in step a) the genipap fruit extract is obtained by grinding the unripe fruit with defined size and texture, respectively, between about 20mm and about 150mm, preferably between about 39 and about 90mm, and between approximately 500gf and approximately 10,000gf, preferably approximately 1,000gf and approximately 6,000gf, under high rotation of about 1,000 to about 5,000 rpm, preferably about 1,800 rpm. The extract is separated from the fruit by centrifugal force, the extract being then cooled down to between 2 and approximately 10°C, preferably approximately 5°C, and filtered in a press or vacuum filter.

Then, the genipap extract is standardized by adding primary amino acid, preferably glycin (following parameters detailed in step c) mentioned before), heated up to the temperature of about 50 to about 95°C, preferably about 80°C, for a time that may vary from about 30 minutes to about 5 hours, preferably about 1 hour.

In said preferred step c), the amino acid may be selected based on the genipin content present, and primary amino acids, among which are, in varying amounts, glutamic acid, serine, glycine, hystidine, arginine, threonine, alanine, proline, tyrosine, valine, methionine, cystine, isoleucine, leucine, phenylanine and lysine, which are the chiefly responsible for the formation of coloration that will give rise to the precipitated insoluble extract.

It is possible to standardize the soluble genipap extract by adding primary amino acids like those cited above, preferably glycine, under heating up to between about 50 and about 90°C, preferably 80°C, for a time of approximately 30 minutes to approximately 5 hours, preferably approximately 1 hour, under stirring. The amount of amino acid to be added ranges from about 0.10 to about 5.00% with respect to the mass of genipap extract and will be established during the standardization for obtaining an extract with final coloration determined in reflectance spectrophotometer, the L*a*b* values of which are between: L* = 10.00 - 50.00, preferably 24.00 +/- 0.30, a* = (-1.00) - 3.00, preferably 0.20+/-0.50 and b* = 0.10 - 2.00, preferably 0.20+/- 0.10. The mordant used in step d) may be selected from:
(1) polyvalent metal salt, which is added as a solution or a dispersion of polyvalent metal salt, comprising polyvalent metals selected from the group consisting of aluminum, iron, zinc, copper, calcium, barium, magnesium, manganese, antimony, tin and strontium. Still more preferably, the solution of polyvalent metal salt comprises calcium or aluminum salts comprising CaCO3 or Al₂(SO4)₃.18H₂O.
   In general, lacquers are produced by adding a substrate and a precipitating agent. Both are classified as mordants. In the case of the insoluble compound produced with genipap extract, the substrate used was calcium carbonate (adsorvent), and the precipitating agent was tannin. Tannin binds to the extract dye, and calcium carbonate adsorbs same. In some cases, other calcium salts are used with precipitating agents binding to the dye. Both the chemical linkage and the adsorption depend on ideal conditions of pH and temperatures, as described herein. The precipitating agents or the adsorbents are selected according to the affinity of each dye, and are not equal in all the processes for obtaining colored insoluble compounds.
(2) Natural substrate, which is added as a solution of natural substrate. The natural substrate solution comprises compounds derived from gallic acid, preferably tannin (an aqueous solution of tannin extract).

In step d), after heating the substrate and still under heating, an aqueous suspension of calcium carbonate is added at approximately 2 to approximately 40%, preferably approximately 9%, by weight based on the total weight of the extract, followed by addition of a solution of hydrated aluminum sulfate, zinc sulfate, copper sulfate, or other polyvalent metal salts like barium, magnesium, manganese, antimony, tin and strontium salts, or other aluminum, zinc and copper salts that are not sulfates, previously acidified with an acid like hydrochloric acid, sulfuric acid and acetic acid, preferably acetic acid, until a range of pH of about 2 to about 6, preferably about 4 to about 5, is reached. The mixture of extract and calcium carbonate should be maintained under heating and stirring for about 30 minutes to about 5 hours, preferably about 1 hour, at a temperature of about 50 to about 96°C, preferably about 80°C.

Preferably, calcium carbonate is used due to its morphology and physicochemical properties. Additionally, another mordent may be used that will act in an adjuvant manner with calcium carbonate. Since calcium carbonate makes the adsorption of the extract possible, calcium carbonate together with another mordent fixes the extract, making it insoluble in polar and/or non-polar media.

After adding calcium carbonate to the extract, another mordent (natural substances or polyvalent metal salts) is added and after obtaining the precipitate in an aqueous medium, the solid substance obtained is filtered under vacuum or in press-filter, by procedures and with equipment usually employed for such purpose.

The solid substance obtained is washed to remove remaining water-soluble extract, and its conductivity is adjusted by the washes themselves or, if necessary, by adding a positive or negative electrolyte that enables one to obtain the desired conductivity between 50mV and 150mV, preferably between 70 and 90mV.

The solid substance obtained, insoluble in polar or non-polar substances, is preferably dried in an oven, at a temperature of about 30 to about 80°C, more preferably of about 50 to about 60°C, until the final moisture reaches about 0 to about 10%, preferably about 1 to about 2%.

The dried solid substance is then ground until its particle size reaches a range of about 15µm to about 150µm, preferably about 30 to about 50µm.

According to a preferred embodiment, the process of the present invention is carried out from natural acacia extract, which contains at least 73% of tanning agents and calcium carbonate.

Soluble substances from genipap-extract precipitate obtainable according to the process of the invention are also embodiments of the present invention, the physicochemical characteristics of which are illustrated ion Table 1 below:

**TABLE 1**

| Physicochemical characteristics | |
|---|---|
| Aspect | Powder |
| Color | Blue |
| pH (10% aqueous solution) | About 4 to about 8 |
| Particle size | About 15 to about 150µm |
| Apparent density (g/cm³) | About 0.2000 - about 0.9500 |
| Total ashes | About 20 - about 80% |

The present invention further relates to compositions comprising the insoluble substances from genipap extract precipitates, obtained by the process described above, and to a carrier or excipient.

The present invention also refers to cosmetic, pharmaceutical, food, textile and paint compositions, which comprise insoluble substances from genipap extract precipitates and a carrier or excipient that, in the case of cosmetic, pharmaceutical, food compositions, is a physiologically acceptable carrier or excipient. The term "physiologically acceptable" should be understood within the broadest scope available in the art, indicating, in general, that the substance in question does not interact, harm or cause side effects to the normal functions of the cells, tissues, organs and systems of healthy living beings.

Said cosmetic, pharmaceutical or food compositions may further comprise active compounds selected from the group consisting of: sunscreens, color agents, emollients, antioxidants, film-forming agents, structure agents, thickeners, wetting agents, pH-adjusting agents, preservatives, sensorial agents, suspending agents, binders, disagglomerating agents, emulsifiers or combinations thereof.

The carriers, excipients and active compounds mentioned above embrace all the substances usually employed in the cosmetic, pharmaceutical, textile, paint and varnish and food industries. By way of example, without restriction of the scope of the invention, the following are cited:
- sunscreens: 2-ethylhexyl methoxycinnamate, titanium dioxide;
- color agents: organic or inorganic pigments;
- emollients: capric/caprylic triglyceride, stearoyl isocetyl, certified natural alpha-bisbolol FSC, dicaprylic ether, essential/plant oils (e.g. castor oil, hydrogenated castor oil); capric/caprylic glycerides, Murumuru butter, decyl oleate, polybutene, isopropyl palmitate;
- antioxidants: tocoferol, ascorbyl palmitate, ascorbic acid, citric acid, butyl hydroxyl toluene;
- structure agents: stearic acid, candelilla wax, carnauba wax, sorbitan olivate, ceresin, glyceryl triberrenine/berrenate;
- thickeners: xanthan gum, stearic acid;
- wetting agents: glycerin, plant glycerin, propylene glycol;
- pH-adjusting agents: triethanolamine;
- preservatives: potassium sorbate, phenoxyethanol, DMDM hydantoin;
- sensorial agents: glyceryl tribehenin/behenate, mica, tapioca starch;
- suspensing agent: propylene stearalconium hectorite/carbonate, coloidal silicon dioxide, aluminum and magnesium silicate;
- binders: synthetic wax, glyceryl tri-behenine/behenate, stearoyl isocetyl;
- disagglomerating agents: colloidal silicon dioxide;
- emulsifiers: glyceryl stearate, sorbitan olivate, cetearyl olivate, sorbitan olivate;
- carrier/excipients: water, plant extracts (e.g. genipap extract), talc.

The cosmetic, pharmaceutical or food compositions according to the present invention may comprise emulsions for face and body and makeup, waxy and oily products, powders, toilet soaps, (aqueous or oily) gels, (aqueous, oily or emulsion) pastes and lotions.

Another embodiment of the invention relates to the use of insoluble substances from genipap extract precipitates according to the present invention in the cosmetic, pharmaceutical, textile, paint and varnish and food industries. Particularly, the invention comprises the use of the insoluble substances from genipap extract precipitates, also an object of the present invention, in preparing cosmetic, pharmaceutical or food compositions, imparting color to the product.

Another embodiment of the present invention is a method for imparting colour to the skin and/or keratinic material of a human or animal, characterised by comprising the topical administration of the insoluble substances from genipap extract precipitates of the present inventions or the cosmetic compositions of the present invention to the human or animal.

### EXAMPLES

For illustrative purposes, which should not be interpreted as being limitative of the scope of the invention, some examples are presented below related to some embodiments of the inventions: (i) process for obtaining substances from genipap extract precipitates; (ii) substances from genipap extract precipitates, and (iii) cosmetic composition containing substances from genipap extract precipitates. Powders like shades, emulsions like eye-lash mascaras and eyeliners, and gels using the genipap extract precipitates according to the present invention were prepared by processes and techniques usual in organic chemistry, cosmetics and pharmacy.

### EXAMPLE 1: - A process for obtaining substances from genipap extract precipitates and substances from genipap extract precipitates:

A process for obtaining substances from genipap extract precipitates were carried out by the steps of:
a) extracting the dye from the unripe genipap fruit previously classified, grinding the fruit under high rotation and separating the juice from the unripe fruit by centrifugal force; the extract was then cooled and filtered;
b) standardizing the genipap extract by adding glycin and heating the genipap extract up to a temperature of about 80°C, for about 1 hour;
c) after heating the extract in step b), one added to the extract, still under heating, an aqueous dispersion of calcium carbonate at 9%, previously acidified with acetic acid, until the pH range of about 4 to about 5 was reached; in this step the mixture of extract and calcium carbonate solution was kept under heating and stirring for about 1 hour, at a temperature of about 80°C;
d) after adding the calcium carbonate to the extract in step c), one added to the extract a mordant comprising an aqueous solution of Al₂(SO4)₃.18H₂O:
   d.1) addition of the aqueous solution of Al₂(SO4)₃.18H₂O: one dissolved the Al₂(SO4)₃-18H₂O at 9% in water, in a pH range of about 0.9 to about 2; one added the solution of Al₂(SO4)₃.18H₂O to the mixture of genipap extract and calcium carbonate and kept under stirring and heating at about 80°C for 1 hour;
e) after obtaining the precipitate in an aqueous medium, the solid substance obtained was filtered under vacuum or in a press-filter, by procedures and with equipment usually employed in the art for this purpose;
f) the solid substance obtained in step d) was washed with demineralized water, and its conductivity was reduced until conductivity values between 50 and 150mV, preferably between 70 and 90mV, were achieved;
g) the solid substance obtained, insoluble in polar or non-polar mediums, was dried in an oven, at a temperature of about 50 to about 60°C, until a final moisture of about 1 to about 2% was reached;
h) the dried solid substance was ground until its particle size reached about 15µm to about 150µm, preferably about 30 to about 50µm.

The powdered solid substances obtained from genipap extract precipitates obtained by the process described above exhibited the physicochemical characteristics described in Table 2 below:

**TABLE 2**

| Physicochemical characteristics | |
|---|---|
| Aspect | Powder |
| color | blue |
| pH (10% aqueous solution) | About 4 to about 8 |
| Particle size | About 15 to about 150µm |
| Apparent density (g/cm3) | About 0.2000 to about 0.9500 |
| Total ashes | About 20 to bout 80% |

### EXAMPLE 2 - A process for obtaining substances from genipap extract precipitates and substances from genipap extract precipitates

- A process for obtaining substances from genipap extract precipitates were carried out by the steps of:
a) extracting the dye from the previously classified genipap unripe fruit, grinding the fruit under high rotation and separating the juice from the unripe fruit by centrifugal force; the extract was then cooled and filtered;
b) standardization of the genipap extract by adding glycin and heating the genipap extract up to a temperature of about 80°C for about 1 hour;
c) after heating the extract in step b), one added to the extract, still under heating, an aqueous dispersion of calcium carbonate at 9%, previously acidified with acetic acid, until the pH range of about 4 to about 5 was reached; in this step the mixture of extract and calcium carbonate solution was kept under heating and stirring for about 1 hour, at a temperature of about 80°C;
a) after adding calcium carbonate to the extract in step c), one added to the extract the mordant comprising an aqueous solution of tannin extract;
   d.1) addition of the aqueous solution of tanin; a 9% tannin extract is dissolved in water at a pH range of 4 to 5 and is added to the mixture of genipap extract and calcium carbonate and kept under stirring and heating at about 80°C for 1 hour;
e) after obtaining the precipitate in an aqueous medium, the solid substance obtained was filtered under vacuum or in press-filter, by the procedures and with the equipment usually employed in the art for this purpose;
f) the solid substance obtained in step d) was washed with demineralized water and its conductivity was reduced until a conductivity values between 50 and 150mV, preferably 70 and 90mV;
g) the solid substance obtained, insoluble in polar or non-polar mediums, is dried in an oven, at a temperature of about 50 to about 60°C until a final moisture of about 1 to about 2% is reached;
h) the dried solid substance is ground until its particle size reaches about 15µm to about 150µm, preferably about 30 - to about 50µm.

The substances powdered solid substances from genipap extract precipitates obtained by the process described above exhibited the physicochemical characteristics described in Table 3 below.

**TABLE 3**

| Physicochemical characteristics | |
|---|---|
| Aspect | Powder |
| Color | Blue |
| pH(10% aqueous solution) | About 4 to about 8 |
| Particle size | Bout 15 to about 150µm |
| Apparent density | About 0.2000 to about 0.9500 |
| Total ashes | About 20 to about 80% |

### EXAMPLE 3 - Eyeliner

| Component | Concentration range | Preferred amount | Function |
|---|---|---|---|
| Genipap extract | 30-70 | 58.750 | Carrier |
| Glycerin | 1.5-6.0 | 4.000 | Wetting agent |
| Propylene glycol | 0.50-3.00 | 2,000 | Wetting agent |
| Aluminum and magnesium silicate | 0.05-1.0 | 0.000 | Suspending agent |
| Xanthan gum | 0.1-1.0 | 0.300 | Thickener |
| Insoluble genipap precipitate obtained according to example 1 or 2 | 0.5-50.0 | 30.000 | Color agent |
| Stearic acid | 0.10-1.00 | 0.500 | Structure agent/thickener |
| Glyceryl stearate | 0.1-0.50 | 0.100 | Emulsifier |
| Sorbitan olivate | 0.1-1.00 | 0.750 | Emulsifier |
| Cetearyl olivate, sorbitan olivate | 1.00-5.00 | 1.000 | Emulsifier |
| Capric/caprylic triglyceride | 0.1-2.00 | 0.500 | Emollient |
| Capric/caprylic triglyceride, stearalconium hectoryl, propylene carbonate | 0.50-0.80 | 0.100 | Suspending agent |
| triethanolamine | 0.05-1.00 | 0.100 | pH adjust |
| Potassium sorbate | 0.05-0.10 | 0.100 | Preservative |
| water | 0.50-1.00 | 1.0000 | Carrier |
| phenoxyethanol | 0.1-1.00 | 0.600 | dye |

### EXAMPLE 4 - Shade

| Component | Concentration range | Preferred amount | Function |
|---|---|---|---|
| Potassium sorbate | 0.50-0.10 | 0.100 | Preservative |
| Synthetic wax | 0,05-1,00 | 0.100 | Binder |
| Talc | 1.00-90.00 | 35.540 | Carrier |
| Colloidal silicon dioxide | 0.005-0.010 | 0.010 | Disagglomerating agent |
| Dlyceryl tri-behenin/behenate | 1.00-10.00 | 4.000 | Sensorial agent |
| PEG-8, tocopherol, ascorbyl palmitate, ascorbic acid and citric acid | 0.1-0.05 | 0.050 | Antioxidant |
| Stearoyl isocetyl stearate | 0.50-10.00 | 4.000 | Emollient/binder |
| Phenyloxyethanol | 0.1-1.0 | 1.000 | Preservative |
| Certificated natural alphabisabolol FSC | 0.05-1.00 | 0.200 | emollient |
| Insoluble genipap precipitate obtained according to example 1 or 2 | 1.00-90.00 | 50.000 | Color agent |

### EXAMPLE 5 - Cream shade

| Component | Concentration range | Preferred amount | Function |
|---|---|---|---|
| PEG-8, tocopherol, ascorbyl palmitate, ascorbic acid and citric acid | 0.05-0.10 | 0.100 | Antioxidant |
| Dicaprylic ether | 1.00-20.00 | 10.000 | Emollient |
| Capric/caprylic triglyceride | 5.00-20.00 | 12.400 | Emollient |
| Castor oil | 2,00-30.00 | 23.500 | Emollient |
| Capric/caprylic glycerides | 0.10-1,00 | 0.500 | Emollient |
| Insoluble genipap precipitate obtained according to example 1 or 2 | 1.00-50.00 | 20.000 | Color agent |
| Tapioca starch | 1.00-25.00 | 20.000 | Sensorial agent |
| Murumuru butter | 1.00-10.00 | 3.000 | Emollient |
| Candelilla wax | 1.00-15.00 | 3.500 | Structure agent |
| Carnauba wax | 1.00-15.00 | 4.000 | Structure agent |
| Sorbitan olivate | 1.100-10.00 | 3.000 | Structure agent |

### EXAMPLE 6 - Gel shade

| Component | Concentration range | Preferred amount | Function |
|---|---|---|---|
| Xanthan gum | 0.10-2.00 | 1.0000 | Thickener |
| Plant glycerin | 0.10-5.00 | 1.0000 | Wetting agent |
| Standardized genipap extract | 10.00-90.00 | 88,4009 | Color agent |
| Demineralized water | 0.50-90.000 | 5.0000 | carrier |
| Triethanolamine | 0.10-1.00 | 0.0000 | pH adjust agent |
| Acrylate copolymer | 1.00-10.00 | 2.0000 | Film-former |
| Potassium sorbate | 0.05-2.00 | 0.10000 | Preservative |
| Demineralized water | 1.00-2.00 | 1.0000 | carrier |
| DMDM hydantoin | 0.50-1.00 | 1.0000 | Preservative |
| Insoluble genipap precipitate obtained according to example 1 or 2 | 1.00-20.00 | 5.0000 | Color agent |

### EXAMPLE 7 - lipstick

| Component | Concentration range | Preferred amount | Function |
|---|---|---|---|
| 2-ethylhexyl methoxycinnamate | 1.00-6.00 | 2.00 | Sunscreen |
| Titanium dioxide | 1.00-4.00 | 1.00 | Sunscreen |
| Pigment | 0.50-10.00 | 2.00 | Color agent |
| Insoluble genipap precipitate obtained according to example 1 or 2 | 0.50-10.00 | 4.00 | Color agent |
| Decyl oleate | 5.00-20.00 | 12.00 | Emollient |

| Castor oil | 20.00-70.00 | 55.15 | Emollient |
|---|---|---|---|
| Tuloene butyl hydroxy | 0.01-0.05 | 0.05 | Antioxidant |
| Glyceryl abietate | 1.00-3.50 | 2.00 | Film former |
| Carnauba wax | 0.50-5.00 | 1.00 | Structure agent |
| Candelilla wax | 0.50-7.00 | 4.00 | Structure agent |
| Ceresine | 0.050-5.00 | 3.50 | Structure agent |
| Hydrogenated castor oil | 0.50-8.00 | 3.30 | Emollient |
| Polybutene | 2.00-10.00 | 7.50 | Emollient |
| Isopropyl palmitate | 1.00-10.00 | 2.50 | Emollient |

## Claims

1. A process for obtaining substances from genipap extract precipitates, **characterized by** comprising the following steps:
a) obtaining an extract from a genipap fruit;
b) adding a mordent to the extract obtained in (a), wherein the mordent is a polyvalent metal salt selected from the group consisting of aluminum, iron, zinc, copper, calcium, barium, magnesium, manganese, antimony, tin and strontium and/or a natural substrate comprising compounds derived from gallic acid, preferably tannin; and
c) separating the solid substances precipitated from step (b).

2. A process according to claim 1, **characterized in that** the polyvalent metal salt comprises calcium or aluminum salts comprising CaCO₃ or Al₂(SO4)₃.18H₂O.

3. A process according to claim 1, **characterized in that**, in step b), calcium carbonate is added as an aqueous dispersion of calcium carbonate.

4. A process according to any one of claims 1 to 3, **characterized in that** step a) comprises the steps:
a1) - obtaining an extract from a genipap fruit by grinding the fruit under high rotation, separating the extract from the fruit, followed by cooling and filtering;
a2) -semi-purifying the raw extract and qualifying non-dyeing compounds;
a3) - standardizing the extract by adding primary amino acids and heating the filtrate obtained up to temperatures in the range of about 50 to about 95°C, for a time that may range from 30 minutes to about 5 hours.

5. A process according to any one of claims 1 to 4, **characterized in that** the step b) comprises the steps:
b1) adding to the extract obtained in a) an aqueous suspension of calcium carbonate in approximately 2 to approximately 40%, preferably approximately 9%, by weight, based on the total weight of the extract, the mixture of extract and calcium carbonate solution being kept under heating and stirring for about 30 minutes to about 5 hours, at a temperature of about 50 to about 95°C;
b2) subsequently adding a mordent comprising a tannin solution, the mixture of extract and tannin solution being kept under heating and stirring for about 30 minutes to about 5 hours, at a temperature of about 50 to about 95°C;
b3) filtering the solid substance obtained, washing, reducing the conductivity and drying at a temperature of about 30 to about 80°C, until the final moisture reaches about 0 to about 10%.

6. A process according to claim 5, **characterized in that** the aqueous solution of calcium carbonate is previously acidified with acetic acid, until reaching a pH range of about 2 to about 6.

7. A process according to any one of claims 1 to 8, **characterized in that** the resulting substance obtained from the precipitation of the genipap extract is insoluble in polar or non-polar substances.

8. Substances from genipap extract precipitates, **characterized in that** they are obtainable by the process as defined in any one of claims 1 to 7.

9. A cosmetic, pharmaceutical or food composition, **characterized by** comprising substances from genipap extract precipitates as defined in claim 8 and a physiologically acceptable carrier or excipient.

10. Use of substances from genipap extract precipitates as defined in claim 8, **characterized in that** it is in the preparation of cosmetic, pharmaceutical or food compositions.

11. A method for imparting colour to the skin and/or keratinic material of a human or animal, **characterized by** comprising the topical administration of substances from genipap extract precipitates as defined in claim 8 or a cosmetic composition as defined in claim 9 to the human or animal.

## Patentansprüche

1. Ein Verfahren zur Gewinnung von Substanzen von Jenipapo-Extrakt-Präzipitaten, **dadurch gekennzeichnet, dass** die folgenden Schritte umfasst sind:
a) Gewinnen eines Extrakts von einer Jenipapo-Frucht,
b) Zugabe eines Beizmittels zu dem in (a) gewonnenen Extrakt, wobei das Beizmittel ein Salz eines mehrwertigen Metalls, ausgewählt aus der aus Aluminium, Eisen, Zink, Kupfer, Calcium, Barium, Magnesium, Mangan, Antimon, Zinn und Strontium bestehenden Gruppe, und/oder ein natürliches Substrat, umfassend von Gallussäure, vorzugsweise Tannin, abgeleitete Verbindungen, ist und
c) Abtrennen der in Schritt (b) präzipitierten festen Substanzen.

2. Verfahren gemäß Anspruch 1, **dadurch gekennzeichnet, dass** das Salz des mehrwertigen Metalls Calcium- oder Aluminiumsalze, umfassend CaCO₃ oder Al₂(SO₄)₃.18H₂O, umfasst

3. Ein Verfahren nach Anspruch 1, **dadurch gekennzeichnet, dass** in Schritt b) Calciumcarbonat als eine wässrige Dispersion von Calciumcarbonat zugegeben wird.

4. Ein Verfahren nach irgendeinem der Ansprüche 1 bis 3, **dadurch gekennzeichnet, dass** der Schritt a) die Schritte umfasst:
a1) - Gewinnen eines Extrakts von einer Jenipapo-Frucht durch Vermahlen der Frucht unter hoher Rotation, Abtrennen des Extrakts von der Frucht, gefolgt von Kühlen und Filtrieren,
a2) - Semireinigen des Rohextrakts und Qualifizieren der nicht-färbenden Verbindungen,
a3) - Standardisieren des Extrakts durch Zugeben primärer Aminosäuren und Erhitzen des erhaltenen Filtrats auf Temperaturen in dem Bereich von etwa 50 bis etwa 95°C während einer Zeit, die zwischen 30 Minuten und etwa 5 Stunden liegen kann.

5. Ein Verfahren nach irgendeinem der Ansprüche 1 bis 4, **dadurch gekennzeichnet, dass** der Schritt b) die Schritte umfasst:
b1) Zugeben einer wässrigen Suspension von Calciumcarbonat in etwa 2 bis etwa 40 Gewichts-%, vorzugsweise etwa 9 Gewichts-%, bezogen auf das Gesamtgewicht des Extrakts, zu dem in a) erhaltenen Extrakt, wobei die Mischung von Extrakt und Calciumcarbonatlösung unter Erhitzen und Rühren während etwa 30 Minuten bis etwa 5 Stunden auf einer Temperatur von etwa 50 bis etwa 95°C gehalten wird,
b2) nachfolgendes Zugeben eines Beizmittels, umfassend eine Tannin-Lösung, wobei die Mischung von Extrakt und Tannin-Lösung unter Erhitzen und Rühren während etwa 30 Minuten bis etwa 5 Stunden auf einer Temperatur von etwa 50 bis etwa 95°C gehalten wird,
b3) Filtrieren der erhaltenen festen Substanz, Waschen, Herabsetzen der Leitfähigkeit und Trocknen bei einer Temperatur von etwa 30 bis etwa 80°C, bis der endgültige Feuchtigkeitsanteil etwa 0 bis etwa 10% erreicht.

6. Ein Verfahren nach Anspruch 5, **dadurch gekennzeichnet, dass** die wässrige Lösung von Calciumcarbonat vorhergehend mit Essigsäure angesäuert wird, bis ein pH-Bereich von etwa 2 bis etwa 6 erreicht ist.

7. Ein Verfahren nach irgendeinem der Ansprüche 1 bis 8, **dadurch gekennzeichnet, dass** die aus der Präzipitation vom Jenipapo-Extrakt erhaltene Substanz in polaren oder nicht-polaren Substanzen unlöslich ist.

8. Substanzen von Jenipapo-Extrakt-Präzipitaten, **dadurch gekennzeichnet, dass** sie durch das Verfahren, wie in irgendeinem der Ansprüche 1 bis 7 definiert, erhältlich sind.

9. Eine kosmetische, pharmazeutische oder Lebensmittel-Zusammensetzung, **gekennzeichnet durch** Einbezug von Substanzen von Jenipapo-Extrakt-Präzipitaten, wie im Anspruch 8 definiert, und eines physiologisch verträglichen Trägers oder Arzneimittelträgers.

10. Verwendung von Substanzen von Jenipapo-Extrakt-Präzipitaten, wie in Anspruch 8 definiert, **dadurch gekennzeichnet, dass** sie zur Herstellung von kosmetischen, pharmazeutischen oder Lebensmittel-Zusammensetzungen verwendet werden.

11. Ein Verfahren, mit dem Haut und/oder keratinisches Material von einem Menschen oder einem Tier Farbe verliehen wird, **gekennzeichnet durch** das Umfassen der topischen Verabreichung von Substanzen von Jenipapo-Extrakt-Präzipitaten, wie im Anspruch 8 definiert, oder einer kosmetischen Zusammensetzung, wie im Anspruch 9 definiert, auf Mensch oder Tier.

## Revendications

1. Procédé pour l'obtention de substances à partir de précipités d'extrait de génipa, **caractérisé en ce qu'**il comprend les étapes suivantes qui consistant :
a) à obtenir un extrait d'un fruit de génipa ;
b) à ajouter un mordant à l'extrait obtenu dans (a), où le mordant est un sel de métal polyvalent choisi dans le groupe consistant en l'aluminium, le fer, le zinc, le cuivre, le calcium, le baryum, le magnésium, le manganèse, l'antimoine, l'étain et le strontium et/ou un substrat naturel comprenant des composés dérivés de l'acide gallique, de préférence le tannin ; et
c) à séparer les substances solides précipitées dans l'étape (b).

2. Procédé selon la revendication 1, **caractérisé en ce que** le sel de métal polyvalent comprend des sels de calcium ou d'aluminium, comprenant du CaCO₃ ou de l'Al₂ (SO4)₃.18H₂O.

3. Procédé selon la revendication 1, **caractérisé en ce que**, dans l'étape b), du carbonate de calcium est ajouté sous forme de dispersion aqueuse de carbonate de calcium.

4. Procédé selon l'une quelconque des revendications 1 à 3, **caractérisé en ce que** l'étape a) comprend les étapes consistant :
a1) - à obtenir un extrait d'un fruit de génipa par broyage du fruit sous une forte rotation, par séparation de l'extrait du fruit, suivie d'un refroidissement et d'un filtrage ;
a2) - à semi-purifier l'extrait brut et à qualifier des composés non-colorants ;
a3) - à standardiser l'extrait en ajoutant des acides aminés primaires et en chauffant le filtrat obtenu jusqu'à des températures se trouvant dans la plage allant d'environ 50°C à environ 95°C, pour une durée pouvant aller de 30 minutes à environ 5 heures.

5. Procédé selon l'une quelconque des revendications 1 à 4, **caractérisé en ce que** l'étape b) comprend les étapes qui consistant :
b1) à ajouter à l'extrait obtenu dans a) une suspension aqueuse de carbonate de calcium en approximativement 2 à approximativement 40 %, de préférence approximativement 9 % en poids, par rapport au poids total de l'extrait, le mélange d'extrait et de solution de carbonate de calcium étant maintenu sous chauffage et agitation pendant environ 30 minutes à environ 5 heures, à une température allant d'environ 50°C à environ 95°C ;
b2) à ajouter ensuite un mordant comprenant une solution de tannin, le mélange d'extrait et de solution de tannin étant maintenu sous chauffage et agitation pendant environ 30 minutes à environ 5 heures, à une température allant d'environ 50°C à environ 95°C ;
b3) à filtrer la substance solide obtenue, à laver, à réduire la conductivité et à sécher à une température allant d'environ 30°C à environ 80°C, jusqu'à ce que l'humidité finale atteigne environ 0 % à environ 10 %.

6. Procédé selon la revendication 5, **caractérisé en ce que** la solution aqueuse de carbonate de calcium est préalablement acidifiée avec de l'acide acétique, jusqu'à ce qu'elle atteigne une plage de pH allant d'environ 2 à environ 6.

7. Procédé selon l'une quelconque des revendications 1 à 8, **caractérisé en ce que** la substance résultante obtenue de la précipitation de l'extrait de génipa est insoluble dans des substances polaires ou non polaires.

8. Substances provenant de précipités d'extrait de génipa, **caractérisées en ce qu'**elles peuvent être obtenues par le procédé tel que défini dans l'une quelconque des revendications 1 à 7.

9. Composition cosmétique, pharmaceutique ou alimentaire, **caractérisée en ce qu'**elle comprend des substances provenant de précipités d'extrait de génipa telles que définies dans la revendication 8 et un support ou excipient physiologiquement acceptable.

10. Utilisation de substances provenant de précipités d'extrait de génipa telles que définies dans la revendication 8, **caractérisée en ce qu'**elle est pour la préparation de compositions cosmétiques, pharmaceutiques ou alimentaires.

11. Procédé pour conférer une couleur à la peau et/ou la matière kératinique d'un être humain ou d'un animal, **caractérisé en ce qu'**il comprend l'administration topique de substances provenant de précipités d'extrait de génipa telles que définies dans la revendication 8 ou d'une composition cosmétique telle que définie dans la revendication 9 à l'être humain ou à l'animal.
